# EUROPEAN PATENT SPECIFICATION

(11) **EP 3 145 336 B1**
(45) Date of publication and mention of the grant of the patent: **12.08.2020**
(21) Application number: 15724195.1
(22) Date of filing: 12.05.2015
(51) Int. Cl.: A23P 10/00, A01K 5/02, A23L 33/00

(54) **SYSTEM, PROCESS AND DEVICE FOR PRODUCING A NUTRITIONAL COMPOSITION WITH PERSONALIZED NUTRIENT CONTENT**
SYSTEM, VERFAHREN UND VORRICHTUNG ZUR HERSTELLUNG EINER ERNÄHRUNGSZUSAMMENSETZUNG MIT PERSONALISIERTEM NÄHRSTOFFGEHALT
SYSTÈME, PROCÉDÉ ET DISPOSITIF DE PRODUCTION D'UNE COMPOSITION NUTRITIONNELLE À TENEUR EN NUTRIMENTS PERSONNALISÉE

(30) Priority: 21.05.2014 EP 14169300
(43) Date of publication of application: 29.03.2017
(73) Proprietor: Société des Produits Nestlé S.A., 1800 Vevey (CH)
(72) Inventor: BAETGE, Edward, CH-1025 St-Sulpice (CH); BOUCHE, Nicolas, CH-1619 Les Paccots (CH); REZZI, Serge André Dominique, CH-1623 Semsales (CH)
(74) Representative: Kamibayashi, Lynne
(86) International application number: PCT/EP2015/060432
(87) International publication number: WO 2015/176994

(56) References cited:
- WO-A1-03/056493
- WO-A1-2005/111955
- US-A1- 2006 099 310
- US-A1- 2011 137 242
- US-A1- 2011 160 902
- US-A1- 2013 034 633

## Description

The present invention relates to the field of nutritional supplementation. In particular, the invention relates to a system, process and device for producing nutritional compositions.

### Background of the invention

The nutritional requirement describes the need of an organism to ingest nutrients, e.g. water, energy, macronutrients (e.g. proteins, carbohydrates, fats) or micronutrients (e.g. minerals, organic acids, trace elements, vitamins), for maintaining vital and other biological functions. Nutritional requirements depend, at least partly, on the metabolic rate of an individual, vary between individuals and life stage, e.g. in men and women, children and adults, elderly people, and are altered under certain conditions, e.g. during pregnancy or breast-feeding, while smoking, or in case of suffering from certain diseases. Hence, certain nutrients can be considered essential or conditionally essential nutrients. For example, cancer, AIDS, rheumatoid arthritis, diabetes, or pancreatitis is known to alter the nutritional requirement of an individual. If, however, the nutritional requirement is not satisfied by nutrient intake over a longer period of time, a condition of malnutrition will develop which, in turn, may adversely affect the progress of disease further.

In both healthy and diseased subjects, routine supplementation of nutrients that are regarded as critical (e.g. vitamins and trace elements) may be advantageous, for instance in terms of promoting health, preventing disease or managing an ongoing condition. However, not only deficiencies in nutrient intake, but also excess of certain nutrients may be detrimental. While, for example, water-soluble vitamins are eliminated by the organism when overdosed, excessive intake of fat-soluble vitamins may lead to hypervitaminosis associated with nausea, vomiting and headache. Also iodide, a trace element, should be handled with care as an overdose of which may lead to thyroid dysfunction.

Accordingly, personalized supplementation of nutrients tailored to the specific requirements of an individual patient would be desirable.

It was thus an object of the present invention to provide methods and products useful in personalized nutritional supplementation.

### Summary of the invention

The aim of the present invention is achieved by subject-matter specified in independent claim 1. Particular embodiments of the invention are as specified in the dependent claims.

The object of the invention is solved by a system for producing a nutritional composition, the system comprising (a) a measurement device for determining a level of one or more nutrients in a sample from a subject; (b) a controller for calculating a required nutrient intake for the subject, wherein the controller is operatively linked to the measurement device and is configured to calculate the required nutrient intake based on the level of the nutrients in the sample from the subject; (c) a nutritional dispenser for preparing a personalized nutritional composition comprising a combination of nutrients required by the subject; wherein the nutritional dispenser is operatively linked to the controller and is configured to produce the personalized nutritional composition comprising the combination of nutrients based on the required nutrient intake for the subject calculated by the controller.

The controller is configured to (i) calculate a difference between a target value and an actual value of the level of the nutrients in the sample; and (ii) calculate a required nutrient intake for the subject based on the difference between the target and actual values.

In one embodiment, the system comprises a feedback loop after delivery of the personalized nutritional composition back to the measurement device to monitor the subject's micronutrient status after receiving the personalized nutrient composition. In one embodiment, the personalized nutritional composition is a printed nutritional composition, preferably a 2D (two-dimensional) printed, an inkjet-printed or 3D (three-dimensional) printed nutritional composition.

In one embodiment, the nutritional composition is for oral administration, preferably for sublingual, perlingual or buccal administration.

In one embodiment, the nutritional composition is provided as an administration form selected from the group consisting of a tablet, a foil, a film, and a wafer.
In one embodiment, the nutritional composition is a food product or an otherwise edible product.

In one embodiment, the measurement device comprises a light spectrometer, a mass spectrometer or NMR spectrometer.

In one embodiment, the nutrient(s) are selected from the group consisting of micronutrients, macronutrients, essential nutrients and phytonutrients.

In one embodiment, at least one nutrient is a micronutrient.

In one embodiment, the micronutrient(s) are selected from the group consisting of fatty acids, amino acids, nucleotides, vitamins, antioxidants, minerals, trace elements, and electrolytes.

In one embodiment, at least one micronutrient is a hydrosoluble vitamin selected from the group consisting of vitamin B₁ (thiamine), vitamin B2 (riboflavin), vitamin B₃ (nicotinic acid or nicotinamide), vitamin B₅ (pantothenic acid), vitamin B6 (pyridoxine, pyridoxal P or pyridoxamine), vitamin B₈ (biotin), vitamin B₉ (folic acid), vitamin B12 (cobalamin) and vitamin C (ascorbic acid).

In another embodiment, at least one micronutrient is a liposoluble vitamin selected from the group consisting of vitamin K₃ (menadione), vitamin K2 (menaquinone), vitamin K₁ (phylloquinone), vitamin E (α-tocopherol or d-tocopherol), vitamin D2 or vitamin D₃.

In another embodiment, at least one micronutrient is a mineral selected from the group consisting of calcium (Ca), chloride (Cl), chromium (Cr), cobalt (Co) as part of Vitamin B12,
copper (Cu), iodine (I), iron (Fe), fluoride (Fl), magnesium (Mg), manganese (Mn), molybdenum (Mo), phosphorus (P), potassium (K), selenium (Se), sodium (Na), sulphur (S), and zinc (Zn).

In another embodiment, at least one micronutrient is an amino acid selected from the group consisting of alanine, arginine monomethylarginine, assymetric-dimethylarginine, symetric-dimethylarginine, asparagine, aspartic acid, citrulline, cystine, cysteine, glutamic acid, glutamine, glycine, histidine, homocysteine, hypotaurine, isoleucine, leucine, lysine, methionine, ornithine, phenylalanine, proline, serine, taurine, threonine, tryptophan, tyrosine and valine.

In another embodiment, at least one micronutrient is a fatty acid selected from C₄:O, C6:0, C8:0, C10:0, C11:0, C12:0, C13:0, C14:0, C15:0, C16:0, C17:0, C18:0, C20:0, C21:0, C22:0, C24:0, C14:1 n-5, C15:1 n-5, C16:1 n-7, C17:1 n-7, C18:1 n-9 trans, C18:1 n-9 cis, C20:1 n-9, C22:1 n-9, C24:1 n-9, C18:2 n-6 trans, C18:2 n-6 cis, C18:3 n-6, C18:3 n-3, C20:2 n-6, C20:3 n-6, C20:3 n-3, C20:4 n-6, C22:2 n-6, C20:5 n-3 and C22:6 n-3 fatty acids.

In one embodiment, at least one nutrient is a macronutrient.

In one embodiment, the macronutrient(s) is selected from the group consisting of carbohydrates, fats, proteins, amino acids and water.

In one embodiment, at least one nutrient is an essential nutrient or conditionally essential nutrient.

In one embodiment, at least one nutrient is a phytonutrient.

In another embodiment, the phytonutrient(s) are selected from the group consisting of terpenoids (isoprenoids) such as carotenoids, triterpenoid, monoterpenes and steroids; phenolic compounds, for example natural monophenols, polyphenols (e.g. flavonoids, isoflavonoid, flavonolignan, lignans, stilbenoids, curcuminoids, stilbenoid and hydrolysable tannin); aromatic acids (e.g. phenolic acids and hydroxycinnamic acids); capsaicin; phenylethanoids; alkylresorcinols; glucosinolates; betalains and chlorophylls.

In one embodiment, the nutritional dispenser comprises a 3D (three-dimensional) food printer.

In a further aspect, the invention may be used in a process for producing a nutritional composition, the process comprising (a)determining a level of one or more nutrients in a sample from a subject; (b) calculating a required nutrient intake for the subject, based on the level of the nutrients in the sample from the subject; (c) preparing a personalized nutritional composition for the subject; wherein the personalized nutritional composition comprises a combination of nutrients required by the subject, as calculated based on the level of nutrients in the sample from the subject.

In another aspectof the invention, the process is repeated until the target nutrient intake and the actual nutrient intake converge.

In a further aspect, the invention comprises a device comprising (a) a controller for calculating a required nutrient intake for a subject, wherein the controller is configured to receive data from a measurement device concerning a level of one or more nutrients in a sample from the subject, and to calculate the required nutrient intake based on the level of the nutrients in the sample from the subject; (b) a nutritional dispenser for preparing a personalized nutritional composition comprising a combination of nutrients required by the subject; wherein the nutritional dispenser is operatively linked to the controller and is configured to produce the personalized nutritional composition comprising the combination of nutrients based on the required nutrient intake for the subject calculated by the controller.

### Brief description of the drawings

Figure 1 is a diagrammatic representation of a system according to the present invention.
Figure 2 is a flow chart illustrating one way of carrying out the process of the present invention.

### Detailed description of the invention

The present invention provides a system for producing a nutritional composition with tailored nutrient content, i.e. the content of one or more nutrients is adapted to the specific requirements of an individual subject. In this sense, the invention is regarded as contributing to the increasingly promising field of personalized nutrition.

The present invention allows for individually preparing nutritional compositions on demand and *"in situ",* e.g. at home or in a hospital. In this manner, the delay between detecting a deficiency in nutrient intake and administering a nutritional composition for compensating the deficiency can be kept short. In some embodiments, the invention may provide the nutritional composition as a natural food composition. In some embodiments, the invention may make use of printing technologies such as 2D (two-dimensional) printing, inkjet- or 3D (three-dimensional) printing.

### Nutrients

The term "nutrient" refers to compounds having a beneficial effect on the body e.g. to provide energy, growth or health. The term includes organic and inorganic compounds.

As used herein the term nutrient may include, for example, macronutrients, micronutrients, essential nutrients, conditionally essential nutrients and phytonutrients.

These terms are not necessarily mutually exclusive. For example, certain nutrients may be defined as either a macronutrient or a micronutrient depending on the particular classification system or list. The expression "at least one nutrient" or "one or more nutrients" means, for example, one, two, three, four, five, ten, 20 or more nutrients.

The term "determining a level of one or more nutrients" includes determining metabolites and/or biomarkers of individual nutrients. Thus in some embodiments, a level of e.g. a metabolite or other indicator of one or more of the above nutrients is measured. Metabolites as indicators of nutritional status are described, for example, in Rezzi et al., Trends in Analytical Chemistry 52 (2013): 112-119.

### Macronutrients

The term "macronutrient" is well known in the art and is used herein according to it standard meaning to refer to a nutrient which is required in large amounts for the normal growth and development of an organism.

Macronutrients include, but are not limited to, carbohydrates, fats, proteins, amino acids and water. Certain minerals may also be classified as macronutrients, such as calcium, chloride, or sodium.

### Micronutrients

The term "micronutrient" refers to compounds having a beneficial effect on the body, e.g. to provide energy, growth or health, but which are required in only minor or trace amounts. The term includes both organic and inorganic compounds, e.g. individual amino acids, nucleotides and fatty acids; vitamins, antioxidants, minerals, trace elements, e.g. iodine, and electrolytes, e.g. sodium chloride, and salts thereof.

An illustrative list of vitamins includes, vitamins A, D, E, K, B1, B2, B6, B12, and C, retinol, retinyl acetate, retinyl palmitate, beta-carotene, cholecalcipherol, ergocalcipherol, D-alpha-tocopherol, DL-alpha-tocopherol, D-alpha-tocopheryl acetate, D-alpha-tocopheryl acid succinate, phyllochinone, thiamine hydrochloride, thiamine mononitrate, riboflavin, sodium riboflavin-5'-phospate, nicotinic acid, nicotinamide, calcium-D-pantothenate, sodium-d-pantothenate, dexpanthenol, pyridoxine hydrochloride, pyridoxine-5'-phosphate, pyridoxine dipalmitate, pteroyl-monoglutamic acid, cyancobalamin, hydroxocobalamin, D-biotin, L-ascorbic acid, sodium-L- ascorbate, calcium-L-ascorbate, potassium-L-ascorbate, and L-ascorbyl-6-palmitate.

An illustrative list of minerals includes calcium (Ca), chloride (Cl), chromium (Cr), cobalt (Co) as part of Vitamin B12, copper (Cu), iodine (I), iron (Fe), fluoride (Fl), magnesium (Mg), manganese (Mn), molybdenum (Mo), phosphorus (P), potassium (K), selenium (Se), sodium (Na), sulphur (S), and zinc (Zn).An illustrative list of organic acids includes, acetic acid, citric acid, lactic acid, malic acid, choline and taurine.

An illustrative list of amino acids includes, L-alanine, L-arginine, L-cysteine, L-histidine, L-glutamine acid, L-glutamine, L-isoleucine, L-leucine, L-lysine, L-methionine, L-ornithine, phenylalanine, L-threonine, L-tryptophan, L- tyrosine, and L-valine.

An illustrative list of fatty acids includes C4:0, C6:0, C8:0, C10:0, C11:0, C12:0, C13:0, C14:0, C15:0, C16:0, C17:0, C18:0, C20:0, C21:0, C22:0, C24:0, C14:1 n-5, C15:1 n-5, C16:1 n-7, C17:1 n-7, C18:1 n-9 trans, C18:1 n-9 cis, C20:1 n-9, C22:1 n-9, C24:1 n-9, C18:2 n-6 trans, C18:2 n-6 cis, C18:3 n-6, C18:3 n-3, C20:2 n-6, C20:3 n-6, C20:3 n-3, C20:4 n-6, C22:2 n-6, C20:5 n-3 and C22:6 n-3 fatty acids. In the nomenclature CX:Y, X refers to the total number of carbon atoms in the fatty acid and Y defines the total number of double bonds in the fatty acid.

### Phytonutrient

The term "phytonutrient" refers to a bioactive plant-derived compound associated with positive health effects.

An illustrative, non-exhaustive list of phytonutrients includes: terpenoids (isoprenoids) such as carotenoids, triterpenoid, monoterpenes and steroids; phenolic compounds, for example natural monophenols, polyphenols (e.g. flavonoids, isoflavonoid, flavonolignan, lignans, stilbenoids, curcuminoids, stilbenoid and hydrolysable tannin); aromatic acids (e.g. phenolic acids and hydroxycinnamic acids); capsaicin; phenylethanoids; alkylresorcinols; glucosinolates; betalains and chlorophylls.

### Essential nutrient

The term "essential nutrient" is used herein to refer to a nutrient which the subject cannot synthesize endogenously, or cannot synthesize at the level required for good health. For example an essential nutrient may be a nutrient which must be obtained from the subject's diet.

An illustrative, non-exhaustive list of essential nutrients includes essential fatty acids, essential amino acids, essential vitamins and essential dietary minerals.

Essential amino acids for humans include phenylalanine, valine, threonine, tryptophan, methionine, leucine, isoleucine, lysine and histidine.

Essential fatty acids for humans include alpha-linolenic acid and linoleic acid.

In addition, the nutrient may be "conditionally essential" depending on, for example, whether the subject has a specific disease, condition or genotype.

### Sample

In general the term "sample" as used herein refers to any body fluid or other tissue sample types, e.g. blood, plasma, serum, sputum, saliva, sweat (perspiration) or urine. Techniques for obtaining such samples from subjects are well known. The term also includes samples of other tissues or fluids obtained by contact with body tissues, e.g. exhaled breath or contact with the skin.

The present method is typically practiced outside of the human or animal body, e.g. on a body fluid sample that was previously obtained from the subject to be tested. Preferably the sample is derived from blood, i.e. the sample comprises whole blood or a blood fraction such as blood plasma or serum.

Techniques for collecting blood samples and separating blood fractions are well known in the art. For instance, vena blood samples can be collected from patients using a needle and deposited into plastic tubes. The collection tubes may, for example, contain spray-coated silica and a polymer gel for serum separation. Serum can be separated by centrifugation at 1300 RCF for 10 min at room temperature and stored in small plastic tubes at -80°C.

### Measurement device

The system comprises a measurement device. The device may comprise any suitable device for determining a level of a nutrient in a sample from a subject. For example, the measurement may be any type of analytical device or system, for instance a spectroscopy device, for example a light spectrometry system, a mass spectrometry system, high-resolution NMR spectroscopy system, etc. Various suitable methods are described in, for example, Rezzi et al., Trends in Analytical Chemistry 52 (2013):112-119.

In one embodiment, the measurement device may be a biosensor. A biosensor is an analytical device, used for the detection of the nutrient and combines a biological component with a physicochemical detector. A biosensor typically consists of a bio-recognition component, biotransducer component, and electronic system which include a signal amplifier, processor, and display. Transducers and electronics can be combined, e.g., in CMOS-based microsensor systems. The recognition component, often called a bioreceptor, uses biomolecules from organisms or receptors modeled after biological systems to interact with the nutrient of interest. This interaction is measured by the biotransducer which outputs a measurable signal proportional to the presence of the target nutrient in the sample. The general aim of the design of a biosensor is to enable quick, convenient testing at the point of care where the sample was procured.

### High-resolution NMR spectroscopy

In one embodiment, the measurement device comprises an NMR spectrometer. NMR spectroscopy offers the unique prospect to profile holistically hundreds of nutrients and/or their metabolites with no a priori selection in an analytically robust manner and with no or very limited sample preparation (see e.g. F.P. Martin et al., Magn. Reson. Chem. 49 (2011) S47-S54; J.C. Lindon et al., Annu. Rep. NMR Spec. 38 (1999) 1-88). In some embodiments, parallel analysis of urine and blood plasma nutrient profiles may be performed. Intact tissue samples can also be profiled by high-resolution magic angle spinning NMR spectroscopy using minimal sample preparation. Proton NMR spectroscopy may be used for sensitivity reasons, while the carbon-13 nucleus can also be measured, often for confirming molecular identity or even for structure elucidation purposes using multidimensional techniques.

### Mass spectrometry (MS)

In another embodiment, the measurement device comprises a mass spectrometer. MS may be employed for global or targeted profiling, e.g. as described in I.D. Wilson et al., J. Chromatogr. B Analyt. Technol. Biomed. Life Sci. 817 (2005) 67-76 and M.R. Wenk, Nat. Rev. Drug Discov. 4 (2005), 594-610. MS can be coupled to gas chromatography (GC) or liquid chromatography (LC), including at the nL scale, to enable highly sensitive metabolite analysis using a range of ionization techniques, but requiring preliminary sample preparation. A broad panel of methods is available for measuring classes of various nutrients and their metabolites, such as amino acids, fatty acids, organic acids, vitamins and phytonutrients. Thanks to recent technological advances, MS analytical performance in terms of sensitivity, mass accuracy, scan rate and resolution improved to the point of allowing profiling of biological samples even in the absence of a preliminary chromatographic step, such as in lipid analysis (i.e. lipidomics) (see K. Schuhmann et al., J. Mass Spectrom. 47 (2012) 96-104.

Thus in particular embodiments, the mass spectrometer may use an ionization method selected from electron impact (El), chemical ionization (Cl), field ionization (FDI), electrospray ionization (ESI), laser desorption ionization (LDI), matrix assisted laser desorption ionization (MALDI) and surface enhanced laser desorption ionization (SELDI). In further embodiments, the mass spectrometry detection method is selected from quadrapole mass spectroscopy (QMS), fourier transform mass spectrometry (FT-MS) and time-of-flight mass spectrometry (TOF-MS).

The measurement device is operatively linked to the controller, e.g. the measurement device and controller are in electronic communication with one another. In particular, the measurement device provides data concerning the level of the nutrients to the controller for further processing. In some embodiments, data concerning the subject (e.g. age, sex, weight or other criteria) may also be provided to the controller. In some embodiments, the measurement device may communicate with the controller via a telecommunications or data network, e.g. via the internet or a wireless or mobile communications system, including short-range radio technologies. For instance, suitable communications protocols include TCP/IP, CDMA, GSM, GPRS, EDGE, Bluetooth, Inmarsat, WLAN, ISDN and so on.

### Controller

The system further comprises a controller for calculating a required nutrient intake for the subject. The controller is operatively linked to the measurement device and is configured to calculate the required nutrient intake based on the level of the nutrients in the sample from the subject.

The controller may be implemented in hardware and/or software, and typically comprises a microprocessor. The controller is configured to perform calculations based on data received from the measurement device, e.g. via a communications network.

For instance, the controller may be configured to (i) calculate a difference between a target value and an actual value of the level of the nutrients in the sample; and (ii) calculate a required nutrient intake for the subject based on the difference between the target and actual values.

The term "target value of nutrient intake", as used in the claims, is linked to the nutritional requirement of an individual. The nutritional requirement may be given as the amount of nutrient [e.g. in g, mg, µg, ng, pg, or mol] per time interval, preferably per 24 hours or one day. Further time intervals (e.g. minute, hour, week, month) are also considered. If nutrient intake occurs only once a day, the "target value of nutrient intake" may be given as the amount of nutrient per day.

Mostly, however, nutrient intake will occur more often than once a day, so that the daily nutritional requirement could be satisfied by more than one meal. In this case, the daily nutritional requirement would be split up. Then, the "target value of nutrient intake" may be given as the amount of nutrient per meal.

The controller may be configured to access one or more databases containing information relating to nutrient requirements. For instance, the controller may access a database of target values of nutrients, e.g. nutrient levels which are indicative of healthy subjects based on criteria such as age, sex, weight and so on. The target values may be in the form of specific values or a range of values indicative of healthy subjects. By comparing the target values from such a database with actual values of nutrient levels, the controller can determine whether the subject requires supplementation with particular nutrients.

The controller may also access a database providing information defining required nutrient supplementation levels. For instance, after the processor calculates a difference between the target and actual values of a nutrient, the processor may access a database which provides an indication of the nutrient intake required in order to restore the nutrient level to the target (e.g. healthy) level. The information in such a database may be based on criteria such as the age, sex, weight and so on of the subject.

The controller is operatively linked to the nutritional dispenser, e.g. the nutritional dispenser and controller are in electronic communication with one another. In particular, the controller provides data concerning the required nutrient intake for the subject to the nutritional dispenser.

In some embodiments, the nutritional dispenser and the controller may be implemented in a single device. In an alternative embodiment, the nutritional dispenser may communicate with the controller via a telecommunications or data network, e.g. via the internet or a wireless or mobile communications system, including short-range radio technologies. For instance, suitable communications protocols include TCP/IP, CDMA, GSM, GPRS, EDGE, Bluetooth, Inmarsat, WLAN, ISDN and so on.

### Nutritional dispenser

The system further comprises a dispenser for producing a nutritional composition. The dispenser may combine various nutrients in varying amounts into a single composition, which is personalized according to the needs of the individual subject.

The nutritional dispenser is able to receive information from the controller regarding the desired amount of at least one nutrient for which the subject is determined to be deficient. Specifically, the nutritional dispenser may receive an input regarding the required nutrient intake for the subject and to incorporate the desired amounts of nutrients into an appropriate composition.

In some embodiments, the nutritional dispenser may comprise a 2D, 3D or inkjet printer.

The term "2D-printing" refers to the process of depositing an essentially two-dimensional composition onto a surface.

The term "inkjet-printing technology" is a type of computer printing that involves propelling droplets of a printing solution or ink onto a support. In case of an "inkjet-printed tablet, foil, film, or wafer", the nutritional composition is inkjet-printed onto an appropriate support. Preferably, the support is soluble in oral fluid, e.g. saliva.

The "3D printing technology" involves a process of making a three-dimensional solid object of virtually any shape from a digital model. Building up the solid object is realized by means of an additive process in which successive layers of material are laid down in the same or different shapes. In case of a "3D printed tablet, foil, film, or wafer", the nutritional composition is included in the tablet, foil, film, or wafer. This may be achieved by mixing the nutritional composition with compounds for forming a matrix (of the administration form), while the resulting mixture must be suitable for 3D printing. Alternating layers with and without nutrients are also considered.

One example of a 3D food printer suitable for use as the nutritional dispenser of the present invention is described in US 2013/034633. Thus in one embodiment the nutritional dispenser comprises an apparatus as described in US 2013/0034633, which is adapted to receive an input regarding the required nutrient intake for the subject. US 2013/0034633 relates to the layer-by-layer prototyping of a three-dimensional (3-D) object from input digital data, specifically the production of an edible food product in this manner. This system involves the freeform fabrication of a food object in a layer manufacturing manner without object specific tooling or human intervention. In accordance with one embodiment, edible food material(s) are distributed layer by layer, and edible binder is selectively ejected upon each successive layer, according to CAD data for the product being formed. Selected regions of the current cross-section are thus fused to previously fused cross-sections. Unbound food material(s) act to support the food product during the fabrication process, allowing for the generation of delicate and intricate food products. Selective colour, flavour, and/or texture may be independently modulated throughout the body of the 3-D food object.

In embodiments of the present invention, the apparatus described in US 2013/0034633 is used to produce a product comprising the desired amount of a mix of specific nutrients, based on the required nutrient intake for the subject determined by the controller.

In another embodiment, the nutritional dispenser may be a device as described in EP2292126A1. EP2292126A1 relates to an apparatus comprising a number of storage hopers for powdered ingredients; a controllable outlet connected to each storage hopper and operable to release controlled amounts of the powdered ingredients; a controllable liquid supply; a mixer; and a control system including a user interface for capturing data from a user. In embodiments of the present invention, the powdered ingredients comprise nutrients. In contrast to the device described in EP2292126A1, according to the present invention the device is operatively connected to a controller and thereby to a measurement device for nutrients, and the personalized nutritional composition is produced based on data relating to nutrient levels in the subject. As such, the required nutrient intake is transmitted to the nutritional dispenser which combines specific quantities of specific powdered ingredients in order to create the desired nutritional composition. In this embodiment, the nutritional dispenser may send signals to the liquid supply and the outlets to release the determined quantity of liquid and each powdered ingredient into the mixer, before dispensing the composition into a container.

In a further embodiment the nutritional dispenser may be a device as described in WO 03/056493. WO 03/056493 describes an apparatus comprising a database arrangement, a user interface and a nutrition and/or medication dispenser which includes rooms for storing different nutrition and/or medical substances. The nutrition dispenser is arranged to define the optimal dose of nutrition and/or medication intended for the person consuming the dose, with the ingredients, amounts and proportions of nutrients at least partly defined with the help of the database arrangement. The apparatus may also comprise the equipment for measuring out the defined nutrients. In embodiments of the present invention, the controller may comprise a database arrangement and processor based on the arrangement as described in WO 03/056493, e.g. capable of receiving an input and determining the required amount of specific nutrients from defined stores to incorporate into the nutritional composition. However, in contrast to the device described in WO 03/056493, according to the present invention the device is operatively connected via the controller to a measurement device for nutrients, and thus the nutrition dispenser produces the personalized nutritional composition based on measurements of nutrient levels and a required nutrient intake for the subject. The nutritional composition comprising a defined mix of nutrients is then produced and dispensed by the apparatus.

In another embodiment the nutrition dispenser may be a device as described in US 2011/0137242, which is directed to a system for preparing a personalized mix of nutrient and supplement ingredients based on the genetic profile of a subject. The system comprises an input device to input genetic information of a user into the system; an identifying device to identify a genetic profile based on the user input information; a processing device to process the information inputted by the user and identify a mix and concentration of ingredients stored in the system and a mixing device to mix the customised mix of nutrient and supplement ingredients to deliver to the user in the form of a beverage. Thus in embodiments of the present invention, the controller and nutrition dispenser may comprise a processing device and mixing device based on those as described in US 2011/0137242. However in contrast to the device described in US 2011/0137242, according to the present invention the device is operatively connected via the controller to a measurement device for nutrients, and thus the nutrition dispenser produces the personalized nutritional composition based on nutrient measurements and a required nutrient intake for the subject. Thus in the present invention, the controller uses this type of input to select the correct mix and concentrations of nutrient ingredients in order to generate a personalised nutritional composition, for example a beverage, for the subject.

In yet another embodiment, the nutrition dispenser may comprise a device as described in WO 2005/111955. WO 2005/111955 discloses a system for dispensing a customised nutritional serving made up of ingredients stored within a device incorporated in the system. The device comprises an ingredient storage module, an ingredient processing module and a serving dispenser. In addition the device comprises a customer interface and a controller operably linked to the user interface and programmed to control the operation of the storage module, the processing module and the dispenser. In use, a customer selects a customised serving through the interface and the controller looks up the information stored in its memory to formulate a serving which best matches the serving selected by the customer, based on predetermined constraints, the controller then directs the mixing of the required ingredients from the ingredient storage module in the processing module and dispenses the serving to the customer. In embodiments of the present invention, the controller and nutritional dispenser may be arranged in a similar manner to the controller, processing module and dispenser as described in WO 2005/111955. However in contrast to the method described in WO 2005/111955, in embodiments of the present invention the nutritional dispenser is configured to receive information relating to the required nutrient intake for the subject from the controller, and to thereby dispense the nutritional composition based on measurements of nutrient levels from a sample from the subject.

In another embodiment, the nutrition dispenser may be based on a device for preparing an individualized dosage form for a patient as described in WO 2005/053608. WO 2005/053608 describes a system comprising three databases storing records associated with the medication needs of individual patients; manufacturing, excipient and packaging details of medicaments; and capsule formulations of pellets, the pellets comprised of medicaments and excipients. A processor is associated with the first, second and third database in order to receive input identifying a specific user and select the required capsules or pellets to meet the medication needs of that user. The selected pellets are then combined in a dispensing station within the apparatus before being presented to the individual. In embodiments of the present invention, the controller and nutritional dispenser may be arranged in a similar manner to the processor and dispensing station as described in WO 2005/053608. However in contrast to the method described in WO 2005/053608, in embodiments of the present invention the nutritional dispenser is configured to receive information relating to the required nutrient intake for the subject from the controller, and to dispense thereby a nutritional composition comprising a mix of nutrients based on measurements of nutrient levels from a sample from the subject. Thus in the present invention, the controller may access databases storing records relating to target levels of particular nutrients for subjects, as well as e.g. the required nutrient intake for subjects based on particular levels of nutrient deficiency. The controller may thereby indicate to the nutritional dispenser the amounts of particular nutrients to combine in order to generate a personalized nutritional composition comprising at least one nutrient for which the subject is deficient.

In yet another embodiment the nutritional dispenser may be an implantable device which is able to generate the required level of nutrients for which the subject is deficient. The implanted dispenser may be configured to receive information relating to the nutrient requirement for the subject from the controller, and to dispense thereby a nutritional composition comprising a mix of nutrients based on measurements of nutrient levels from a sample from the subject.

### Nutritional composition

The nutritional composition may be in the form of an administration unit. The term "administration unit" refers to a unit, optionally consisting of several sub-units, by which the dose of nutrient, i.e. the amount corresponding to the difference between the target value and actual value of nutrient levels, is delivered to the subject.

In some embodiments, the administration unit comprising the nutritional composition does not need to be swallowed or ejected from the mouth. Thus, the administration unit may disintegrate quickly upon placement in the subject's mouth, thereby releasing the nutrients into oral fluid, so that the nutrients are swallowed with oral fluid and absorbed in the digestive tract (enteral absorption). For that purpose, the administration unit and the nutritional composition may be based on water-soluble material, in particular on material soluble in oral fluid such as saliva. In addition or alternatively, the nutrients are at least partly absorbed by the oral mucosa (parenteral absorption).

A "foil" or a "film" means a thin, flexible leaf or sheet of a layer of a material. Optionally, more than one layer of the same or different material may be provided (multi-layered foil or film).

In some embodiments the personalized nutritional composition may be a natural complex assembly of nutrients in their natural, native matrix from foodstuffs

In another embodiment, the personalized nutritional composition may comprise protein and/or lipid carriers which facilitate delivery of the nutrients.

The nutritional composition may be a beverage, tablet, capsule, film, ink-jet produced or 3D-printing produced product.

The nutritional composition may be in a form which is suitable for topical administration. For example the composition may be a lotion, a cream or an ointment.

The nutritional composition may be a food mix or food product which provides a mix of nutrients based on measurements of nutrient levels from a sample from the subject. The nutritional composition may be in the form of a food product or an otherwise edible product.

### Subjects

The subjects may comprise infants, children, adults and elderly people. The subject may be healthy or suffering from a disease, e.g. the subject may be a normal healthy subject, or a nursing home resident and/or a bed-ridden person. The term may also comprise animals, in particular companion animals such as a cat or dog.

Further advantages and features of the present invention will be apparent to those of skill in the art from the following examples along with attached figures.

### Examples

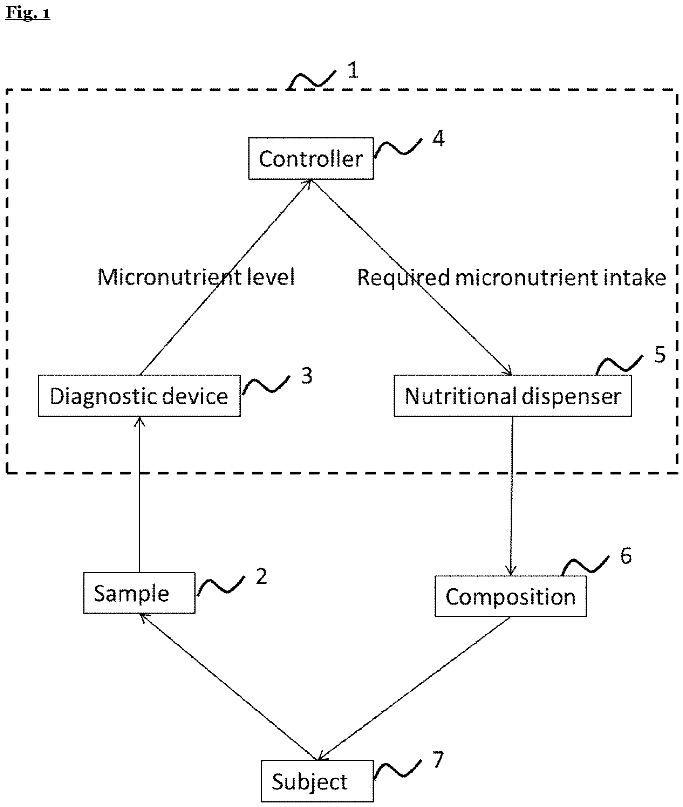

Figure 1 shows one embodiment of a system **1** according to the present invention. The system comprises a diagnostic measurement device **3**, a controller **4** and a nutritional dispenser **5**. The diagnostic measurement device **3** is operatively linked to the controller **4** and the controller **4** is operatively linked to the nutritional dispenser **5**.

In operation, a sample **2** is obtained from the subject **1**. The sample may be, for example, a blood, urine or other tissue sample from the subject. The diagnostic measurement device **3** (e.g. a mass spectrometer) measures a level of one or more nutrients in the sample, and transmits data regarding the nutrient levels in the subject to the controller **4**, for instance using a communications network.

The controller **4** processes the nutrient data from the subject, and may determine whether the levels of particular nutrients are above or below a target value for the subject. For instance, the controller may access a database containing target levels for particular nutrients in healthy subjects, and compare the target levels to the actual levels measured in the subject. By calculating a difference between the target levels and actual levels of each nutrient, the controller determines a required nutrient intake for the subject, e.g. by accessing a second database containing information relating to nutrient supplementation levels.

The controller **4** then transmits information regarding the required nutrient intake for the subject to the nutritional dispenser **5**, for instance using a communications network. The nutritional dispenser **5** may be, for example, a 3D food printer or other device which is capable of producing a defined and specific combination of nutrient ingredients.

The nutritional dispenser **5** receives the information regarding the required nutrient intake for the subject and produces a personalized nutritional composition 6 based thereon. Thus the nutritional dispenser **5** includes in the composition 6 a particular level of each nutrient, according to whether the level of the nutrient in the subject is above or below the target level.

The personalized nutritional composition 6 is then consumed by the subject. In some embodiments, a further sample **2** may be taken, and nutrient levels measured once again. Typically the administration of the personalized nutritional composition should lead to a convergence between the target values and actual values of each nutrient in the subject. If there is a still a significant difference between the target and actual values, a further nutritional composition may be produced and administered to the subject. Accordingly, in some embodiments the method may be performed iteratively or repeatedly until the target and actual values converge. In other embodiments the method may be performed continuously or in regular cycles, in order to maintain the target and actual values within as close as possible a range indefinitely.

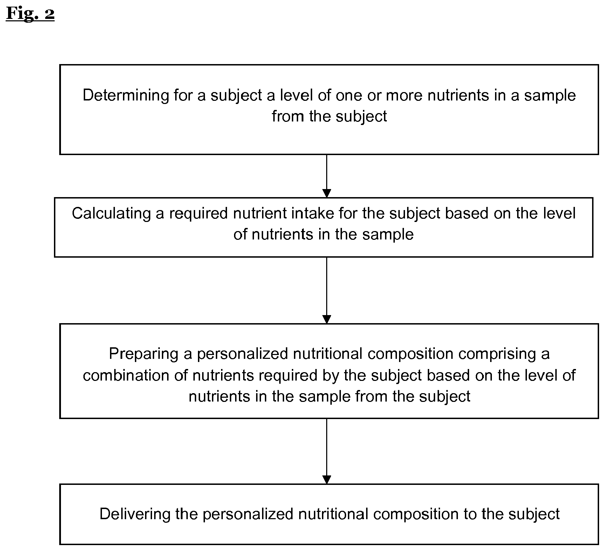

Figure 2 shows one example of an exemplary process according to the present invention.

### Step 1: Determination of nutrient levels in a sample from a subject

The nutritional status of a subject is determined by measurement of nutrient levels, e.g. by mass spectrometry. Determined nutrient levels are transmitted to the controller, e.g. using a communications network, together with data concerning the age, sex and weight of the subject.

### Step 2: Calculating a required nutrient intake for the subject

The controller calculates a required nutrient intake for the subject, based on the nutrient levels transmitted from the detection device. The calculation and quantification steps are executed with the assistance of a data processing system, e.g. a computer.

### Step 3: Manufacture of an inkjet-printedfoil or film

A nutritional composition in the form of a printing solution having a predetermined concentration (amount/volume, e.g. g/l, mg/ml, mol/l, or mmol/l) of a nutrient is prepared. Then, a predetermined volume of the nutritional composition, and thus the desired amount of nutrient, is printed on a foil or a film serving as a support. The process of printing can be carried out in a single printing step or, alternatively, by repeating the step of printing several times.

### Step 4: Manufacture of a 3D printed administration form

A 3D printing solution containing a predetermined concentration of nutrient is prepared. Then, a three-dimensional object is build up by successively layering predetermined volumes of the printing solution, thereby creating an administration form containing the desired amount of nutrient.

## Claims

1. A system (1) for producing a nutritional composition (6), the system (1) comprising:
(a) a measurement device (3) for determining a level of one or more nutrients in a sample (2) from a subject (7);
(b) a controller (4) for calculating a required nutrient intake for the subject, wherein the controller (4) is operatively linked to the measurement device (3) and is configured to calculate the required nutrient intake based on the level of the nutrients in the sample (2) from the subject (7);
(c) a nutritional dispenser (5) for preparing a personalized nutritional composition (6) comprising a combination of nutrients required by the subject (7);
wherein the nutritional dispenser (5) is operatively linked to the controller (4) and is configured to produce the personalized nutritional composition (6) comprising the combination of nutrients based on the required nutrient intake for the subject (7) calculated by the controller (4) and the controller (4) is configured to (i) calculate a difference between a target value and an actual value of the level of the nutrients in the sample; and (ii) calculate a required nutrient intake for the subject (7) based on the difference between the target and actual values.

2. A system (1) according to claim 1 wherein said system (1) comprises a feedback loop after delivery of the personalized nutritional composition (6) back to the measurement device (3) to monitor the subject's nutrient status to ensure that the target value and the actual value of the level of the one or more nutrients converge.

3. A system (1) according to any preceding claim, wherein the nutritional dispenser (5) is , preferably a 2D food printer, an inkjet-printer or 3D food printer suitable for producing the nutritional composition (6).

4. A system (1) according to any preceding claim, wherein the nutritional dispenser (5) is adapted to produce a nutritional composition (6) suitable for oral administration, preferably for sublingual, perlingual or buccal administration.

5. A system (1) according to any preceding claim, wherein the nutritional dispenser (5) is adapted to produce a nutritional composition (6) in the administration form selected from the group consisting of a tablet, a foil, a film, and a wafer.

6. A system (1) according to any preceding claim, wherein the nutritional dispenser (5) Z is adapted to produce a nutritional composition (6) which is a food product or an otherwise edible product.

7. A system (1) according to any preceding claim, wherein the measurement device (3) comprises a light spectrometer, a mass spectrometer or NMR spectrometer.

8. A system (1) according to any preceding claim, wherein the measurement device (3) measures nutrient(s) selected from the group consisting of micronutrients, macronutrients, essential nutrients, conditionally essential nutrients and phytonutrients.

9. A system (1) according to claim 8 wherein the measurement device (3) measures at least one nutrient which is a micronutrient.

10. A system (1) according to claim 8 or claim 9, wherein the measurement device (3) measures micronutrient(s) selected from the group consisting of fatty acids, amino acids, nucleotides, vitamins, antioxidants, minerals, trace elements, and electrolytes.

11. A system (1) according to any of claims 8 to 10, wherein the measurement device (3) measures at least one micronutrient wherein said micronutrient is a hydrosoluble vitamin selected from the group consisting of vitamin B1 (thiamine), vitamin B2 (riboflavin), vitamin B3 (nicotinic acid or nicotinamide), vitamin B5 (pantothenic acid), vitamin B6 (pyridoxine, pyridoxal P or pyridoxamine), vitamin B8 (biotin), vitamin B9 (folic acid), vitamin B12 (cobalamin) and vitamin C (ascorbic acid).

12. A system (1) according to any of claims 8 to 10, wherein the measurement device (3) measures at least one micronutrient wherein said micronutrient is a liposoluble vitamin selected from the group consisting of vitamin K3 (menadione), vitamin K2 (menaquinone), vitamin K1 (phylloquinone), vitamin E (α-tocopherol or d-tocopherol), vitamin D2 or vitamin D3.

13. A system (1) according to any of claims 8 to 10, wherein the measurement device (3) measures at least one micronutrient wherein said micronutrient is a mineral selected from the group consisting of calcium, chloride, chromium, cobalt, copper, iodine, iron, fluoride, magnesium, manganese, molybdenum, phosphorus, potassium, selenium, sodium, sulphur, and zinc.

14. A system (1) according to any of claims 8 to 10 , wherein the measurement device (3) measures at least one micronutrient wherein said micronutrient is an amino acid selected from the group consisting of alanine, arginine monomethylarginine, assymetric-dimethylarginine, symetric-dimethylarginine, asparagine, aspartic acid, citrulline, cystine, cysteine, glutamic acid, glutamine, glycine, histidine, homocysteine, hypotaurine, isoleucine, leucine, lysine, methionine, ornithine, phenylalanine, proline, serine, taurine, threonine, tryptophan, tyrosine and valine.

15. A system (1) according to any of claims 8 to 10, wherein the measurement device (3) measures at least one micronutrient wherein said micronutrient is a fatty acid selected from C4:0, C6:0, C8:0, C10:0, C11:0, C12:0, C13:0, C14:0, C15:0, C16:0, C17:0, C18:0, C20:0, C21:0, C22:0, C24:0, C14:1 n-5, C15:1 n-5, C16:1 n-7, C17:1 n-7, C18:1 n-9 trans, C18:1 n-9 cis, C20:1 n-9, C22:1 n-9, C24:1 n-9, C18:2 n-6 trans, C18:2 n-6 cis, C18:3 n-6, C18:3 n-3, C20:2 n-6, C20:3 n-6, C20:3 n-3, C20:4 n-6, C22:2 n-6, C20:5 n-3 and C22:6 n-3 fatty acids.

16. A system (1) according to claim 8 wherein the measurement device (3) measures at least one nutrient wherein said nutrient is preferably a macronutrient.

17. A system (1) according to claim 8 or claim 16 wherein the measurement device (3) measures the macronutrient selected from the group consisting of carbohydrates, fats, proteins, amino acids and water.

18. A system (1) according to any of claims 8 to 17 wherein the measurement device (3) measures at least one nutrient wherein said nutrient is an essential nutrient.

19. A system (1) according to any of claims 8 to 18 wherein the measurement device (3) measures at least one nutrient wherein said nutrient is a phytonutrient.

20. A system (1) according to claim 19 wherein the measurement device (3) measures the phytonutrient selected from the group consisting of terpenoids (isoprenoids) such as carotenoids, triterpenoid, monoterpenes and steroids; phenolic compounds, for example natural monophenols, polyphenols (e.g. flavonoids, isoflavonoid, flavonolignan, lignans, stilbenoids, curcuminoids, stilbenoid and hydrolysable tannin); aromatic acids (e.g. phenolic acids and hydroxycinnamic acids); capsaicin; phenylethanoids; alkylresorcinols; glucosinolates; betalains and chlorophylls.

## Patentansprüche

1. System (1) zum Herstellen einer Nährstoffzusammensetzung (6), wobei das System (1) Folgendes umfasst:
(a) eine Messvorrichtung (3) zum Bestimmen eines Gehalts von einem oder mehreren Nährstoffen in einer Probe (2) von einem Subjekt (7);
(b) eine Steuerung (4) zum Berechnen einer für das Subjekt erforderlichen Nährstoffaufnahme, wobei die Steuerung (4) betriebswirksam mit der Messvorrichtung (3) verbunden und dazu konfiguriert ist, die erforderliche Nährstoffaufnahme basierend auf dem Gehalt an Nährstoffen in der Probe (2) von dem Subjekt (7) zu berechnen;
(c) einen Nährstoffspender (5) zum Herstellen einer personalisierten Nährstoffzusammensetzung (6), die eine Kombination aus für das Subjekt (7) erforderlichen Nährstoffen umfasst;
wobei der Nährstoffspender (5) betriebswirksam mit der Steuerung (4) verbunden und dazu konfiguriert ist, die personalisierte Nährstoffzusammensetzung (6) herzustellen, die die Kombination aus Nährstoffen basierend auf der für das Subjekt (7) erforderlichen Nährstoffaufnahme, die von der Steuerung (4) berechnet wurde, umfasst, und wobei die Steuerung (4) dazu konfiguriert ist, (i) einen Unterschied zwischen einem Zielwert und einem tatsächlichen Wert des Gehalts an Nährstoffen in der Probe zu berechnen; und (ii) eine für das Subjekt (7) erforderliche Nährstoffaufnahme basierend auf dem Unterschied zwischen dem Zielwert und dem tatsächlichen Wert zu berechnen.

2. System (1) nach Anspruch 1, wobei das System (1) eine Rückkopplungsschleife nach der Abgabe der personalisierten Nährstoffzusammensetzung (6) zurück an die Messvorrichtung (3) umfasst, um den Nährstoffzustand des Subjekts zu überwachen, um sicherzustellen, dass der Zielwert und der tatsächliche Wert des Gehalts an dem einen oder den mehreren Nährstoffen sich annähern.

3. System (1) nach einem der vorstehenden Ansprüche, wobei der Nährstoffspender (5) vorzugsweise ein 2D-Nahrungsmitteldrucker, ein Tintenstrahldrucker oder ein 3D-Nahrungsmitteldrucker ist, der zum Herstellen der Nährstoffzusammensetzung (6) geeignet ist.

4. System (1) nach einem der vorstehenden Ansprüche, wobei der Nährstoffspender (5) dazu ausgelegt ist, eine Nährstoffzusammensetzung (6) herzustellen, die zur oralen Verabreichung, vorzugsweise zur sublingualen, perlingualen oder bukkalen Verabreichung, geeignet ist.

5. System (1) nach einem der vorstehenden Ansprüche, wobei der Nährstoffspender (5) dazu ausgelegt ist, eine Nährstoffzusammensetzung (6) in der Verabreichungsform herzustellen, die ausgewählt ist aus der Gruppe bestehend aus einer Tablette, einer Folie, einem Film und einer Oblate.

6. System (1) nach einem der vorstehenden Ansprüche, wobei der Nährstoffspender (5) dazu ausgelegt ist, eine Nährstoffzusammensetzung (6) herzustellen, bei der es sich um ein Lebensmittelprodukt oder ein anderweitig essbares Produkt handelt.

7. System (1) nach einem der vorstehenden Ansprüche, wobei es sich bei der Messvorrichtung (3) um ein Lichtspektrometer, ein Massenspektrometer oder ein NMR-Spektrometer handelt.

8. System (1) nach einem der vorstehenden Ansprüche, wobei die Messvorrichtung (3) einen oder mehrere Nährstoffe misst, die ausgewählt sind aus der Gruppe bestehend aus Mikronährstoffen, Makronährstoffen, bedingt essenziellen Nährstoffen und Phytonährstoffen.

9. System (1) nach Anspruch 8, wobei die Messvorrichtung (3) mindestens einen Nährstoff misst, bei dem es sich um einen Mikronährstoff handelt.

10. System (1) nach Anspruch 8 oder Anspruch 9, wobei die Messvorrichtung (3) einen oder mehrere Mikronährstoffe misst, die ausgewählt sind aus der Gruppe bestehend aus Fettsäuren, Aminosäuren, Nukleotiden, Vitaminen, Antioxidantien, Mineralien, Spurenelementen und Elektrolyten.

11. System (1) nach einem der Ansprüche 8 bis 10, wobei die Messvorrichtung (3) mindestens einen Mikronährstoff misst, wobei es sich bei dem Mikronährstoff um ein wasserlösliches Vitamin handelt, das ausgewählt ist aus der Gruppe bestehend aus Vitamin B1 (Thiamin), Vitamin B2 (Riboflavin), Vitamin B3 (Nicotinsäure oder Nicotinamid), Vitamin B5 (Panthotensäure), Vitamin B6 (Pyridoxin, Pyridoxalphosphat oder Pyridoxamin), Vitamin B8 (Biotin), Vitamin B9 (Folsäure), Vitamin B12 (Cobalamin) und Vitamin C (Ascorbinsäure).

12. System (1) nach einem der Ansprüche 8 bis 10, wobei die Messvorrichtung (3) mindestens einen Mikronährstoff misst, wobei es sich bei dem Mikronährstoff um ein fettlösliches Vitamin handelt, das ausgewählt ist aus der Gruppe bestehend aus Vitamin K3 (Menadion), Vitamin K2 (Menachinon), Vitamin K1 (Phyllochinon), Vitamin E α-Tocopherol or d-Tocopherol), Vitamin D2 oder Vitamin D3.

13. System (1) nach einem der Ansprüche 8 bis 10, wobei die Messvorrichtung (3) mindestens einen Mikronährstoff misst, wobei es sich bei dem Mikronährstoff um ein Mineral handelt, das ausgewählt ist aus der Gruppe bestehend aus Calcium, Chlorid, Chrom, Cobalt, Kupfer, lod, Eisen, Fluorid, Magnesium, Mangan, Molybdän, Phosphor, Kalium, Selen, Natrium, Schwefel und Zink.

14. System (1) nach einem der Ansprüche 8 bis 10, wobei die Messvorrichtung (3) mindestens einen Mikronährstoff misst, wobei es sich bei dem Mikronährstoff um eine Aminosäure handelt, die ausgewählt ist aus der Gruppe bestehend aus Alanin, Arginin, Monomethylarginin, asymmetrischem Dimethylarginin, symmetrischem Dimethylarginin, Asparagin, Asparaginsäure, Citrullin, Cystin, Cystein, Glutaminsäure, Glutamin, Glycin, Histidin, Homocystein, Hypotaurin, Isoleucin, Leucin, Lysin, Methionin, Ornithin, Phenylalanin, Prolin, Serin, Taurin, Threonin, Tryptophan, Tyrosin und Valin.

15. System (1) nach einem der Ansprüche 8 bis 10, wobei die Messvorrichtung (3) mindestens einen Mikronährstoff misst, wobei es sich bei dem Mikronährstoff um eine Fettsäure handelt, die ausgewählt ist aus den folgenden Fettsäuren: C4:0, C6:0, C8:0, C10:0, C11:0, C12:0, C13:0, C14:0, C15:0, C16:0, C17:0, C18:0, C20:0, C21:0, C22:0, C24:0, C14:1 n-5, C15:1 n-5, C16:1 n-7, C17:1 n-7, C18:1 n-9 trans, C18:1 n- 9 cis, C20:1 n-9, C22:1 n-9, C24:1 n-9, C18:2 n-6 trans, C18:2 n-6 cis, C18:3 n-6, C18:3 n-3, C20:2 n-6, C20:3 n-6, C20:3 n-3, C20:4 n-6, C22:2 n-6, C20:5 n-3 und C22:6 n-3.

16. System (1) nach Anspruch 8, wobei die Messvorrichtung (3) mindestens einen Nährstoff misst, wobei es sich bei dem Nährstoff vorzugsweise um einen Makronährstoff handelt.

17. System (1) nach Anspruch 8 oder Anspruch 16, wobei die Messvorrichtung (3) den Makronährstoff misst, der ausgewählt ist aus der Gruppe bestehend aus Kohlenhydraten, Fetten, Proteinen, Aminosäuren und Wasser.

18. System (1) nach einem der Ansprüche 8 bis 17, wobei die Messvorrichtung (3) mindestens einen Nährstoff misst, wobei es sich bei dem Nährstoff um einen essenziellen Nährstoff handelt.

19. System (1) nach einem der Ansprüche 8 bis 18, wobei die Messvorrichtung (3) mindestens einen Nährstoff misst, wobei es sich bei dem Nährstoff um einen Phytonährstoff handelt.

20. System (1) nach Anspruch 19, wobei die Messvorrichtung (3) den Phytonährstoff misst, der ausgewählt ist aus der Gruppe bestehend aus Terpenoiden (Isoprenoiden) wie Carotinoiden, Triterpenoid, Monoterpenen und Steroiden; Phenolverbindungen, beispielsweise natürlichen Monophenolen, Polyphenolen (z. B. Flavonoide, Isoflavonoid, Flavonolignan, Lignane, Stilbenoide, Curcuminoide, Stilbenoid und hydrolysierbare Tannine); aromatischen Säuren (z. B. Phenolsäuren und Hydroxyzimtsäuren); Capsaicin; Phenylethanoiden; Alkylresorcinolen; Glucosinolaten; Betalainen und Chlorophyllen.

## Revendications

1. Système (1) pour produire une composition nutritionnelle (6), le système (1) comprenant :
(a) un dispositif de mesure (3) pour déterminer un taux d'un ou plusieurs nutriments dans un échantillon (2) provenant d'un sujet (7) ;
(b) un contrôleur (4) pour calculer un apport requis en nutriments pour le sujet, dans lequel le contrôleur (4) est en liaison fonctionnelle avec le dispositif de mesure (3) et est configuré pour calculer l'apport requis en nutriments sur la base du taux des nutriments dans l'échantillon (2) provenant du sujet (7) ;
(c) un distributeur nutritionnel (5) pour préparer une composition nutritionnelle personnalisée (6) comprenant une combinaison de nutriments requis par le sujet (7) ;
dans lequel le distributeur nutritionnel (5) est en liaison fonctionnelle avec le contrôleur (4) et est configuré pour produire la composition nutritionnelle personnalisée (6) comprenant la combinaison de nutriments sur la base de l'apport requis en nutriments pour le sujet (7) calculé par le contrôleur (4) et le contrôleur (4) est configuré pour (i) calculer une différence entre une valeur cible et une valeur réelle du taux des nutriments dans l'échantillon ; et (ii) calculer un apport requis en nutriments pour le sujet (7) sur la base de la différence entre les valeurs cible et réelle.

2. Système (1) selon la revendication 1 dans lequel ledit système (1) comprend une boucle de rétroaction après distribution de la composition nutritionnelle personnalisée (6) qui revient vers le dispositif de mesure (3) pour surveiller l'état de nutriments du sujet pour s'assurer que la valeur cible et la valeur réelle du taux du ou des nutriments convergent.

3. Système (1) selon une quelconque revendication précédente, dans lequel le distributeur nutritionnel (5) est, de préférence une imprimante alimentaire 2D, une imprimante à jet d'encre ou une imprimante alimentaire 3D appropriée pour produire la composition nutritionnelle (6).

4. Système (1) selon une quelconque revendication précédente, dans lequel le distributeur nutritionnel (5) est conçu pour produire une composition nutritionnelle (6) appropriée pour administration orale, de préférence pour administration sublinguale, perlinguale ou buccale.

5. Système (1) selon une quelconque revendication précédente, dans lequel le distributeur nutritionnel (5) est conçu pour produire une composition nutritionnelle (6) sous la forme d'administration choisie dans le groupe constitué d'un comprimé, une feuille, un film, et une gaufrette.

6. Système (1) selon une quelconque revendication précédente, dans lequel le distributeur nutritionnel (5) est conçu pour produire une composition nutritionnelle (6) qui est un produit alimentaire ou un produit autrement comestible.

7. Système (1) selon une quelconque revendication précédente, dans lequel le dispositif de mesure (3) comprend un spectromètre à lumière, un spectromètre de masse ou un spectromètre RMN.

8. Système (1) selon une quelconque revendication précédente, dans lequel le dispositif de mesure (3) mesure un ou des nutriment(s) choisi(s) dans le groupe constitué de micronutriments, macronutriments, nutriments essentiels, nutriments conditionnellement essentiels et phytonutriments.

9. Système (1) selon la revendication 8 dans lequel le dispositif de mesure (3) mesure au moins un nutriment qui est un micronutriment.

10. Système (1) selon la revendication 8 ou la revendication 9, dans lequel le dispositif de mesure (3) mesure un ou des micronutriment(s) choisi(s) dans le groupe constitué d'acides gras, acides aminés, nucléotides, vitamines, antioxydants, minéraux, oligoéléments et électrolytes.

11. Système (1) selon l'une quelconque des revendications 8 à 10, dans lequel le dispositif de mesure (3) mesure au moins un micronutriment dans lequel ledit micronutriment est une vitamine hydrosoluble choisie dans le groupe constitué de vitamine B1 (thiamine), vitamine B2 (riboflavine), vitamine B3 (acide nicotinique ou nicotinamide), vitamine B5 (acide pantothénique), vitamine B6 (pyridoxine, pyridoxal P ou pyridoxamine), vitamine B8 (biotine), vitamine B9 (acide folique), vitamine B12 (cobalamine) et vitamine C (acide ascorbique).

12. Système (1) selon l'une quelconque des revendications 8 à 10, dans lequel le dispositif de mesure (3) mesure au moins un micronutriment dans lequel ledit micronutriment est une vitamine liposoluble choisie dans le groupe constitué de vitamine K3 (ménadione), vitamine K2 (ménaquinone), vitamine K1 (phylloquinone), vitamine E (a-tocophérol ou d-tocophérol), vitamine D2 ou vitamine D3.

13. Système (1) selon l'une quelconque des revendications 8 à 10, dans lequel le dispositif de mesure (3) mesure au moins un micronutriment dans lequel ledit micronutriment est un minéral choisi dans le groupe constitué de calcium, chlorure, chrome, cobalt, cuivre, iode, fer, fluorure, magnésium, manganèse, molybdène, phosphore, potassium, sélénium, sodium, soufre et zinc.

14. Système (1) selon l'une quelconque des revendications 8 à 10, dans lequel le dispositif de mesure (3) mesure au moins un micronutriment dans lequel ledit micronutriment est un acide aminé choisi dans le groupe constitué d'alanine, arginine monométhylarginine, diméthylarginine asymétrique, diméthylarginine symétrique, asparagine, acide aspartique, citrulline, cystine, cystéine, acide glutamique, glutamine, glycine, histidine, homocystéine, hypotaurine, isoleucine, leucine, lysine, méthionine, ornithine, phénylalanine, proline, sérine, taurine, thréonine, tryptophane, tyrosine et valine.

15. Système (1) selon l'une quelconque des revendications 8 à 10, dans lequel le dispositif de mesure (3) mesure au moins un micronutriment dans lequel ledit micronutriment est un acide gras choisi parmi les acides gras C4:0, C6:0, C8:0, C10:0, C11:0, C12:0, C13:0, C14:0, C15:0, C16:0, C17:0, C18:0, C20:0, C21:0, C22:0, C24:0, C14:1 n-5, C15:1 n-5, C16:1 n-7, C17:1 n-7, C18:1 n-9 trans, C18:1 n-9 cis, C20:1 n-9, C22:1 n-9, C24:1 n-9, C18:2 n-6 trans, C18:2 n-6 cis, C18:3 n-6, C18:3 n-3, C20:2 n-6, C20:3 n-6, C20:3 n-3, C20:4 n-6, C22:2 n-6, C20:5 n-3 et C22:6 n-3.

16. Système (1) selon la revendication 8 dans lequel le dispositif de mesure (3) mesure au moins un nutriment dans lequel ledit nutriment est de préférence un macronutriment.

17. Système (1) selon la revendication 8 ou la revendication 16 dans lequel le dispositif de mesure (3) mesure le macronutriment choisi dans le groupe constitué de glucides, graisses, protéines, acides aminés et eau.

18. Système (1) selon l'une quelconque des revendications 8 à 17 dans lequel le dispositif de mesure (3) mesure au moins un nutriment dans lequel ledit nutriment est un nutriment essentiel.

19. Système (1) selon l'une quelconque des revendications 8 à 18 dans lequel le dispositif de mesure (3) mesure au moins un nutriment dans lequel ledit nutriment est un phytonutriment.

20. Système (1) selon la revendication 19 dans lequel le dispositif de mesure (3) mesure le phytonutriment choisi dans le groupe constitué de terpénoïdes (isoprénoïdes) tels que caroténoïdes, triterpénoïde, monoterpènes et stéroïdes ; composés phénoliques, par exemple monophénols naturels, polyphénols (par exemple flavonoïdes, isoflavonoïde, flavonolignane, lignanes, stilbénoïdes, curcuminoïdes, stilbénoïde et tanin hydrolysable) ; acides aromatiques (par exemple acides phénoliques et acides hydroxycinnamiques) ; capsaïcine ; phényléthanoïdes ; alkylrésorcinols ; glucosinolates ; bétalaïnes et chlorophylles.
